# FASCICULE DE BREVET EUROPEEN

(11) **EP 0 346 712 B1**
(45) Date de publication et mention de la délivrance du brevet: **29.12.1993**
(21) Numéro de dépôt: 89110110.7
(22) Date de dépôt: 05.06.1989
(51) Int. Cl.: A61B 17/36, A61B 17/32

(54) **Equipement chirurgical**
Chirurgisches Gerät
Surgical equipment

(30) Priorité: 16.06.1988 CH 2331/88
(43) Date de publication de la demande: 20.12.1989
(73) Titulaire: Rausis, Claude F., CH-1950 Sion (CH)
(72) Inventeur: Rausis, Claude F., CH-1950 Sion (CH)
(74) Mandataire: Micheli & Cie

(56) Documents cités:
- EP-A- 0 194 856
- EP-A- 0 258 901
- JP-A-61 103 690
- US-A- 4 676 242

## Description

La présente invention a pour objet un équipement chirurgical permettant d'inciser, de sectionner, de couper, de réséquer et de traiter des tissus vivants humain ou animal par l'utilisation d'un concept de chirurgie dite "atactile", c'est-à-dire sans contact direct entre un objet de travail contondant à l'instar de : scalpel, ciseau ou autre, et les structures tissulaires du patient.

La chirurgie atactile se pratique depuis l'introduction des faisceaux laser en médecine et tout particulièrement le laser-CO₂. En effet, le laser à gaz carbonique, le plus approprié à la chirurgie, permet un travail à distance sans toucher mécaniquement les tissus. Il a prouvé son efficacité expérimentale et clinique par rapport à l'utilisation du bistouri conventionnel :
- son effet hémostasiant sur les petits vaisseaux permet une réduction des suffusions sanguines dans le champ opératoire, facilitant la vision du chirurgien,
- l'annulation de manipulations traumatisantes des tissus, car il agit sans un quelconque contact métallique dans le champ opératoire, avantage capital en oncologie,
- l'obtention d'une meilleure assurance de la stérilité due au travail à distance, associé à la destruction immédiate de germes au point d'impact du faisceau,
- la réduction des douleurs post-opératoires cicatricielles.

Par contre le laser comme bistouri reste superficiel dans son action, au point plus ou moins élargi de focalisation du faisceau. Ce laser-CO₂ ne maîtrise pas les hémorragies des gros troncs vasculaires, comme on les rencontre dans les importants viscères parenchymateux de l'organisme.

Le deuxième exemple de chirurgie atactile se concrétise actuellement avec l'apparition de nouveaux équipements chirurgicaux émettant un jet d'eau plus ou moins fin de même que sous plus ou moins haute pression (cf "Resection of the liver with a water jet", Br. J. Surg. Vol 69 (1982) 93-94 imprimé en Grande-Bretagne).

Cette technique chirurgicale par action à distance du jet d'eau sous pression est particulièrement intéressante pour la préparation - résection de tissu parenchymateux offrant une résistance moindre au jet d'eau par rapport aux structures conjonctives de soutien comme les gros troncs vasculaires.

Ces deux techniques de chirurgie atactile deviennent ainsi complémentaires, si bien que le premier but de la présente invention est la réalisation d'un équipement de chirurgie atactile conciliant les deux effets : laser-CO₂ et jet d'eau sous pression, à la discrétion en un seul élément dans la main du chirurgien et qui de plus permette un traitement pharmacologique du champ opératoire ainsi qu'une visualisation thermographique simultanée de l'effet tant du laser que du jet d'eau sur les tissus opérés.

L'équipement chirurgical selon la présente invention présente les caractéristiques décrites et revendiquées dans ce qui suit.

Le dessin annexé illustre très schématiquement et à titre d'exemple une forme d'exécution de l'équipement chirurgical selon l'invention.

La figure 1 en est une vue générale en perspective.

La figure 2 illustre schématiquement les dispositifs produisant le rayon laser, le jet d'eau et fournissant les informations thermographiques sur le champ opératoire.

La figure 3 illustre schématiquement et partiellement une pièce à main selon l'invention.

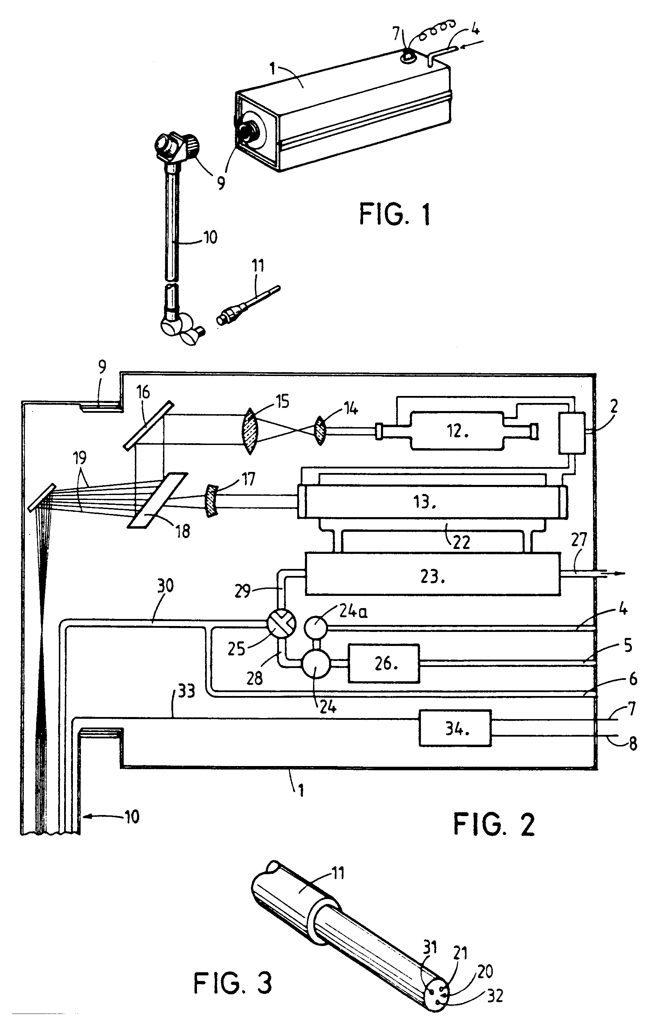

La figure 1 illustre très schématiquement l'équipement chirurgical selon l'invention qui se compose d'un boîtier 1 renfermant le laser de visualisation (12), le laser de travail (13), le dispositif de formation du jet d'eau ainsi que l'équipement électronique de thermographie. Ce boitier 1 dont les différents éléments seront décrits plus en détail ci-après, est équipé d'une part de connexions (2) permettant de le relier à une source électrique de 220 volts alimentant les deux lasers 12,13, à une source de fluide sous pression 4, généralement de l'air, à une d'alimentation d'eau 5, à un conduit d'adjuvants 6, à une source de courant 7 et à une sortie TV 8. D'autre part ce boîtier est équipé d'un connecteur multiple 9 permettant de le relier à un bras articulé ou à un câble flexible 10 dont l'extrémité est munie d'une pièce à main 11. Ce bras flexible ou câble souple 10 permet d'acheminer du boîtier 1 à la sortie de la pièce à main les faisceaux des lasers de visualisation et de travail, le jet d'eau ainsi que la liaison de la sonde thermographique à son équipement électronique d'alimentation et d'analyse.

Se référant plus particulièrement à la figure 2 maintenant, on voit que le boîtier renferme un premier laser de visualisation au HeNe 12 et un second laser de travail au CO₂ 13 dont les faisceaux sont dirigés à l'aide de lentilles 14,15 et d'un miroir 16 respectivement d'une lentille 17 sur un miroir commun 18. Ainsi à la sortie du boîtier 1 les faisceaux visibles du laser 12 et invisibles du laser de travail 13 sont strictement combinés ou superposés et peuvent être focalisés sur un même point ce qui permet à l'opérateur de voir le point d'impact et le degré de focalisation du faisceau du laser de travail 13 en observant visuellement celui du laser de visualisation 12. Ce faisceau combiné 19 est conduit par des miroirs à travers le bras articulé 10 ou par une fibre optique (non illustrée) à travers un câble souple jusqu'à la face frontale 20 de la pièce à main 11 dont il émerge par une ouverture 21.

Les deux lasers 12 et 13 sont du type scellé, le laser CO₂ de travail 13 devant fournir une énergie non négligeable présente une puissance telle qu'il est nécessaire de prévoir un circuit de refroidissement de ce laser. Ce circuit de refroidissement comprend schématiquement un circuit 22 entourant le tube laser 13 branché en circuit fermé sur le primaire d'un échangeur de chaleur 23 dont le secondaire est relié à une pompe de circulation 24, entraînée par un moteur à air comprimé 24a, et par l'intermédiaire d'une vanne à trois voies 25 à un réservoir 26 de liquide de refroidissement, alimenté en liquide par un conduit 5, et à une sortie d'évacuation 27 pouvant être reconnectée en circuit fermé de réalimentation au réservoir 26. Ainsi lorsque la vanne à trois voies 25 relie les conduits 28 et 29 la chaleur émise par le fonctionnement du laser de travail 13 peut être évacuée.

Dans une variante le laser CO₂ peut être refroidi à l'air, dans ce cas le circuit 22 serait directement relié au circuit d'air comprimé 4.

La pompe 24 entraînée par un moteur à air 24a alimenté par la conduite d'air comprimé 4 par l'intermédiaire d'une vanne à trois voies alimenterait directement en eau le conduit 30.

Grâce à cette disposition astucieuse il est possible à l'aide de la même pompe 24, lorsque la vanne à trois voies est dans son autre position reliant le conduit 28 à un conduit 30, d'alimenter en eau sous pression la buse 31 située à l'extrémité de la pièce à main 11, le conduit 30 circulant à l'intérieur du bras articulé 10 ou du câble flexible le remplaçant. Ainsi en utilisant les mêmes éléments, vanne 25 et réservoir d'eau 26, il est possible soit de refroidir le laser de travail 13 lorsqu'il est en fonction, soit de former le jet d'eau servant de bistouri atactile.

Lorsque le jet d'eau est en fonction, il est prévu de pouvoir incorporer par le conduit 6 des adjuvants, médicamenteux de toutes sortes, qui permettent d'avoir une action non seulement mécanique du jet sur les tissus mais également pharmacologique et thérapeutique.

Les commandes contrôlant le fonctionnement des lasers 12,13 de la pompe 24 et de la vanne 25 peuvent soit être groupées sur la pièce à main 11 soit sur un pédalier accessible à l'opérateur. La pièce à main 11 est encore équipée d'une sonde thermographique 32 reliée par un câble électrique 33 passant dans le bras articulé 10 à un équipement électronique 34 situé dans le boîtier et dont la sortie 8 peut être branchée sur un écran TV pour donner en direct l'image thermographique des tissus sur lesquels le chirurgien travaille.

Ceci est très important car les tissus sains et ceux pathologiques, notamment ceux d'une tumeur cancéreuse, n'ont pas le même pouvoir calorifique et peuvent être ainsi distingués les uns des autres par leur réaction d'échauffement provoquée soit par l'impact du faisceau laser de travail soit par l'impact du jet d'eau.

Cet équipement chirurgical est unique en ce qu'il permet de regrouper en un seul appareil et dans une seule pièce à main un bistouri au laser CO₂, un scalpel pharmacologique à jet d'eau sous pression et un équipement de visualisation thermographique de la zone opératoire.

Cet équipement est en outre remarquable en ce que le circuit de refroidissement du laser de travail 13 est combiné avec le système de production du jet d'eau ce qui simplifie l'équipement et le rend très maniable, facile à utiliser, et plus économique à réaliser que des appareils séparés.

Le grand avantage de cet équipement chirurgical combiné est que le chirurgien peut à tout moment en cours d'opération travailler avec le bistouri à laser ou le scalpel à jet d'eau et donc adapter la technique chirurgicale atactile en fonction de la qualité des tissus à sectionner ou à traiter. Il peut en outre en modulant la pression du jet d'eau l'utiliser pour laver le champ opératoire et y apporter des agents pharmacologiques spécifiques, tels : anesthésiants, chimiothérapiques, aseptisants, hémostasiants, etc., ce qui constitue un apport extrêmement utile et important dans la plupart des interventions chirurgicales d'aujourd'hui.

## Revendications

1. Equipement chirurgical (1) pour la chirurgie atactile comprenant un laser de travail (13) et un laser de visualisation (12) dont les faisceaux sont superposés; un circuit de refroidissement (22, 23) du laser de travail (13) dont le fluide est mis en circulation par une source d'air comprimé (24a); cette même source (24a) entraînant une pompe à eau (24) alimentant un scalpel à jet d'eau comportant une buse (31) émettant un jet d'eau sous pression; les faisceaux des lasers et le conduit alimentant ladite buse étant réunis dans un bras (10) souple ou articulé relié à la pièce à main (11).

2. Equipement chirurgical selon la revendication 1, caractérisé par le fait que le circuit de refroidissement (22, 23) du laser de travail (13) comprend un échangeur de chaleur (23) dont le secondaire (23) est parcouru par un liquide mis en circulation par la pompe (24) entraînée par un moteur à air comprimé (24a) alimenté par ladite source; cette même pompe (24) alimentant, par l'intermédiaire d'une vanne à trois voies (25), soit ledit circuit secondaire de l'échangeur, soit le conduit (30) menant à la buse (31) émettant le jet d'eau.

3. Equipement selon la revendication 1, caractérisé par le fait que la source d'air comprimé (24a) alimente, par l'intermédiaire d'une vanne à trois voies (25), soit le circuit de refroidissement (22, 23) du laser de travail (13), soit le moteur entraînant la pompe à eau (24).

4. Equipement selon l'une des revendications précédentes, caractérisé par le fait qu'il comporte encore un réservoir (26) en amont de la pompe à eau.

5. Equipement selon l'une des revendications précédentes, caractérisé par le fait que le conduit d'alimentation (30) de la buse (31) par la pompe est relié à un conduit (6) permettant d'incorporer au liquide pompé des adjuvants pharmacologiques.

6. Equipement selon l'une des revendications précédentes, caractérisé par le fait que la pièce à main (11) est munie d'une sonde thermographique (32) reliée par une liaison électrique (33) passant dans le bras (10) souple ou articulé à un équipement électronique (34) dont la sortie délivre des signaux permettant d'alimenter un écran de visualisation (8) de thermographie donnant une image du champ opératoire réagissant sous l'impact soit du faisceau laser soit du jet d'eau.

7. Equipement selon l'une des revendications précédentes, caractérisé par le fait que les commandes des lasers, du jet d'eau, et de l'introduction d'adjuvants sont regroupés, soit sur la pièce à main (11), soit sur un pédalier, soit distribués en partie sur celle-ci et en partie sur un pédalier.

8. Equipement selon l'une des revendications précédentes, caractérisé par le fait que la pièce à main (11) est reliée par un câble flexible (10) au boîtier (1) renfermant le laser de visualisation (12), le laser de travail (13), les moyens de circulation (22, 23) et de mise sous pression (24a) du liquide ou des fluides de refroidissement du laser de travail (13) et du jet d'eau ainsi que l'appareil (34) de traitement des signaux de la sonde thermographique (32); les faisceaux lasers étant conduits par une fibre optique, le liquide alimentant la buse (31) par un tube flexible et la sonde thermographique (32) par des conducteurs électriques (33) souples au travers dudit câble.

## Claims

1. A surgical equipment (1) for atactile surgery including a working laser (13) and a visualization laser (12) of which the two beams are superimposed; a cooling circuit (22, 23) for the working laser (13), the fluid of which is circulated by means of a source of compressed air (24a); this same source (24a) driving a water pump (24) supplying a water jet scalpel provided with a nozzle (31) emitting a jet of water under pressure; the laser beams and the conduit for supplying said nozzle being lodged in a flexible or in an articulated arm (10) connected to the hand piece (11).

2. A surgical equipment according to claim 1, characterized in that the cooling circuit (22, 23) of tile working laser (13) includes a heat exchanger (23), wherein the fluid flowing in the secondary circuit (23) is circulated by the pump (24) driven by a compressed air motor (24a) supplied from said source; this same pump (24) feeding via a three-way valve (25) either said secondary circuit of the exchanger, or the conduit (30) leading to the nozzle (31) emitting the jet of water.

3. An equipment according to claim 1, characterized in that the source of compressed air (24a) supplies via a three-way valve (25) either tile cooling circuit (22, 23) of the working laser (13), or the motor driving the water pump (24).

4. An equipment according to one of the preceding claims, characterized in that it includes a tank (26) upstream of the water pump.

5. An equipment according to one of the preceding claims, characterized in that the supply conduit (30) extending from the pump to the nozzle (31) is connected to a conduit (6), which makes it possible to incorporate pharmaceutical additives into the liquid being pumped.

6. An equipment according to one of the preceding claims, characterized in that the hand piece (11) is provided with a thermographic sensor (32) connected by an electrical connexion (33) extending through the flexible or the articulated arm (10) to an electronic equipment (34), the output of which produces signals which can be used for supplying a thermographic display (8) to generate a picture of the operation field reacting to the impact either of the laser beam or of the water jet.

7. An equipment according to one of the preceding claims, characterized in that the lasers, the water jet and the additives introduction are either controlled from the hand piece (11), or by pedals, or partly from the hand piece and partly by pedals.

8. An equipment according to one of the preceding claims, characterized in that the hand piece (11) is connected by a flexible cable (10) to the housing (1) containing the visualization laser (12), the working laser (13), the means (22, 23) for circulating and the means (24a) and for pressurizing the liquid or fluids for cooling the working laser (13) and for the water jet, as well as the apparatus (34) for processing the signals from the thermographic sensor (32); the laser beams being conducted by an optical fiber, the liquid being supplied to the nozzle (31) by a flexible tube and the thermographic sensor (32) being power supplied by flexible electrical conductors (33), all of which extend in said cable.

## Patentansprüche

1. Chirurgische Einrichtung (1) für die ataktile Chirurgie mit einem Arbeitslaser (13) und einem Visualisationslaser (12) deren Strahlenbündel überlagert sind; einem Kühlungskreislauf (22,23) des Arbeitslaser (13), dessen Flüssigkeit durch eine Druckluftquelle (24a) in Umlauf gesetzt wird; wobei dieselbe Quelle (24a) eine Wasserpumpe (24) antreibt, die ein Wasserstrahlskapell mit einer einen Wasserstrahl unter Druck bildenden Düse (31) versorgt; und wobei die Strahlenbündel der Laser und die die sogenannte Düse versorgende Leitung in einem biegsamen oder beweglich mit dem Handstück (11) verbundenen Arm (10) vereinigt sind.

2. Chirurgische Einrichtung nach Anspruch 1, dadurch gekennzeichnet, dass der Kühlungskreislauf (22,23) des Arbeitslaser (13) einen Wärmeaustauscher (23) aufweist, dessen Sekundärumlauf (23) von einer Flüssigkeit durchflossen wird, die durch die Pumpe (24) in Umlauf gesetzt wird, welche von dem von der sogenannten Quelle versorgten Drucklaufmotor (24a) angetrieben wird; wobei dieselbe Pumpe (24) mittels eines Dreiwegventils (25) entweder den sogenannten Sekundärumlauf des Austausches oder die Leitung (30), welche zu der den Wasserstrahl bildenden Düse (31) führt.

3. Einrichtung nach Anspruch 1, dadurch gekennzeichnet, dass die Druckluftquelle (24a) über ein Dreiwegventil (25) entweder den Kühlungskreislauf (22,23) des Arbeitslaser (13) oder den die Wasserpumpe (24) auftreibenden Motor versorgt.

4. Einrichtung nach einem der vorhergehenden Ansprüche, dadurch gekennzeichnet, dass sie ein Reservoir (26) vor der Wasserpumpe aufweist.

5. Einrichtung nach einem der vorhergehenden Ansprüche, dadurch gekennzeichnet, dass die die Düse (31) über die Pumpe versorgende Leitung (30) an eine Leitung (6) angeschlossen ist, die es erlaubt, der gepumpten Flüssigkeit pharmacologische Zusatzteile zu zufügen.

6. Einrichtung nach einem der vorhergehenden Ansprüche, dadurch gekennzeichnet, dass das Handstück (11) mit einer thermographischen Sonde (32) versehen ist, die an eine elektrische Verbindung (33) geschlossen, welche in dem biegsamen oder gegliederten Arm (10) zu einer elektronischen Einrichtung (34) führt, deren Ausgang Signale liefert, welche einen Thermographie Bildschirm (8) versorgen, der eine Abbildung des Operationsfeldes zeigt, das unter dem Einfluss entweder des Laserstrahlenbündels oder des Wasserstrahls reagiert.

7. Einrichtung nach einem der vorhergehenden Ansprüche, dadurch gekennzeichnet, dass die Betätigungsorgane der Laser, des Wasserstrahls und der Zusatzteile Einführung entweder auf dem Handstück (11) oder einem Fusspedal gruppiert sind, oder aber zum Teil auf dem Handstück und zum Teil auf dem Fusspedal verteilt sind.

8. Einrichtung nach einem der vorhergehenden Ansprüche, dadurch gekennzeichnet, dass das Handstück (11) über ein biegsames Kabel (10) mit dem Gehaüse (1) verbunden ist, welches den Visualisationslaser (12), den Arbeitslaser (13), die Umlaufmittel (22,23) und unter Druck stellenden Mittel (24a) der Flüssigkeit oder der Fluide zur Kühlung des Arbeitslasers (13) oder des Wasserstrahls, sowie den Signalverarbeitungsapparat (34) der thermographischen Sonde (32) enthält; wobei die Laserstrahlen über optische Fibern, die die Düse (31) versorgende Flüssigkeit über eine biegsame Leitung und die thermographische Sonde (32) über biegsame elektrische Leiter (33) mittels des sogenannten Kabels geleitet werden.
